# EUROPEAN PATENT APPLICATION

(11) **EP 1 335 296 A2**
(43) Date of publication of application: **13.08.2003**
(21) Application number: 03000490.7
(22) Date of filing: 11.01.2003
(51) Int. Cl.: G06F 15/00

(54) **Research system with remote access**

(30) Priority: 30.01.2002 US 58583
(71) Applicant: BASF AKTIENGESELLSCHAFT, 67056 Ludwigshafen (DE)
(72) Inventor: Schrof, Wolfgang, Dr., 67271 Neuleiningen (DE); Lorenz, Thomas, Dr., 67117 Limburgerhof (DE); Lehmann, Stephan, Dr., 67067 Ludwigshafen (DE); Hadeler, Joachim, 67227 Frankenthal (DE); Iden, Rüdiger, Dr., 67373 Dudenhofen (DE); Bandara, Upali, Dr., 69181 Leimen (DE)
(74) Representative: Hössle Kudlek & Partner

(57) **Abstract**

A remote research system, comprising: a central laboratory site equipped to be used for chemical research tests; one or several laboratory robots for performing chemical research tests; a central processing unit (CPU); one or several workstations linked to said central processing unit and set up to control said laboratory robots; one or several cameras for detecting actions of said laboratory robots when performing one of said chemical research tests, said one or several cameras being linked to said central processing unit via said one or several workstations; one or several remote user workstations linked to said central processing unit via a computer network and set up to allow remote user access to chemical research tests performed in said central laboratory site, the execution of a test performed being transmitted by said one or several cameras via said central processing unit and said computer network to said one or several remote user workstations.

## Description

### Technical Field

The technical field of this invention is in the area of chemical research and particularly in the area of combinatorial materials science and research. More particularly, the present invention relates to the field of a remote access system to provide chemical research or combinatorial materials research (CMR) possibilities to experts and scientists located remotely from a central laboratory site.

### Background

Combinatorial chemistry has significantly changed the drug discovery process in the pharmaceutical industry, and promises to bring to the market place more drug entities per unit time than ever before. New products need to be brought to the market place rapidly as a result of the increasing competition in the chemical industry. Enormous pressure is put on research and development, as simultaneously more innovative ideas, higher success rates, shorter development times, and lower research and production costs per product are required. This paradigm was first encountered in the pharmaceutical industry, where long development times and high research costs forced the development of new approaches to dramatically accelerate the drug discovery process. These approaches encompass combinatorial chemistry and high-throughput screening (HTS) and represent a powerful research strategy when applied to problems where a large parameter space controls the properties of a product.

The combinatorial process involves the design and synthesis of discovery libraries aimed at efficiently exploring large number of structurally or compositionally diverse compounds thought to be of interest as a result of an understanding of their chemical, physical, and structural properties. Rapid, sensitive measurements of all relevant chemical or physical or application properties of each library member result in the identification of a family of lead compounds with a desired property. These compounds are then optimized by continuously varying the stoichiometries or structures of a more focused set of compounds in a focus library. Materials with the optimum compositions are then synthesized in quantities sufficient for their detailed characterization. Finally, with methods of combinatorial chemical synthesis evolving together with rapid analysis and high-throughput screening, high-capacity information processing and data management becomes challenging and requires appropriate database technology. Combinatorial chemistry methodologies are increasingly being applied to the field of materials science including homogeneous and heterogeneous catalysis and will unquestionably influence and change the way research is carried out in the future. A concise review on combinatorial materials science and the according state of the art is given in "Angewandte Chemie", Int. Ed., 1999, 38, 2494 - 2532.

As a matter of fact, catalyst discovery and optimization can be dramatically accelerated by coupling automated library synthesis with high-throughput screening methods and sophisticated high-capacity informatic systems. In the contrast to conventional research in a "one at a time" or serial fashion provide thorough quality control of the samples that enter the screening process to ensure that accurate data are obtained, combinatorial methods are much faster and render the preparation of relatively large numbers of compounds feasible but often lack control over the purity of the compounds entering the assay screen. Methods intermediate between these two extremes are based on parallel or array syntheses in a spatially addressable format with usually one compound per well, coupled to automated screens.

However, many complex research processes suffer from restricted efficiency because involved researchers and scientists are distributed around the world. Further restrictions are imposed by changes due to continuous restructuring of industrial companies and their research departments. As a consequence, many research projects experience sensitive time delays due to insufficient, indirect and long-lasting process control. A further problem is caused by the fact that combinatorial materials research is very complex and cost-intensive. Costly research projects usually only exist once as it would not be economical to erect another identical research site. As a result, researchers from other sites around the world have to be integrated to benefit from their expertise and their budgets. More and more research projects are therefore organized and mounted as joint projects between two or more companies in order to generate new products of higher value and price. To support these sensitive relations between companies, new efficient methods to share information and to ensure a fast response on unexpected problems have to be found and implemented.

### Summary

The present invention provides a system which is apt to provide remote research capabilities, e.g. remote designing and planning of a research test, remote preparing of a test, remote launching of a test and remote interfering while a test is performed as well as remote evaluation and interpretation of test results. In general, the system according to the invention provides access for remotely located researchers or customers to a central research site and the tests/experiments prepared and performed there.

The system according to the invention comprises a central laboratory site equipped to be used for research tests; one or several laboratory robots for performing research tests; a central processing unit (CPU); one or several workstations linked to said central processing unit and set up to control said laboratory robots; one or several cameras for detecting actions of said laboratory robots when performing one of said research tests, said one or several cameras being linked to said central processing unit via said one or several workstations; one or several remote user workstations linked to said central processing unit via a computer network and set up to allow remote user access to research tests performed in said central laboratory site, the execution of a test performed being transmitted by said one or several cameras via said central processing unit and said computer network to said one or several remote user workstations. The system according to the invention is particularly suited for use with chemical research tests, whereby in the context of this application the term chemical research test is to be understood as covering all kind of chemical basic research tests as well as physical, physiochemical and application rests.

According to an alternative embodiment, the system comprises a central laboratory site equipped to be used for chemical research tests; a central processing unit (CPU); one or several workstations linked to said central processing unit and set up to control said chemical research tests; a database for storing data relevant to said chemical research tests, said database being connected to said central processing unit; detector elements for automatically detecting results of said chemical research tests; one or several remote user workstations linked to said central processing unit via a computer network and set up to allow remote user access to chemical research tests performed in said central laboratory site, enabling remotely located researchers using said remote user workstations to participate remotely in any stage of the performance one of said chemical research tests.

The system of the invention also is a computer system which is set up to perform remote chemical research and comprising a central server system; a backend database server connected to said central server system; one or several local control computers connected to said central server system and providing access to said central server system for local users; chemical laboratory equipment connected to said one or several local control computers; one or several remote client systems connected to said central server system via a computer network and providing access to said central server system for remotely located users; said central server system further comprising an application server interface for connecting local users and remotely located users to research test services provided by said central server system, said research test services being database services and conceptional services.

The system as proposed by the present invention disposes with the need to gather experts at a laboratory research site at a given location in the world as the remote research system according to the invention provides easy access to the central research site from any point of the world. Experts can be integrated in any stage of a research test, starting from early design and preparation stages to implementation and launching of a test and ending with the evaluation and interpretation of test results.

Linkage between the central site CPU or central server and the remote work stations is provided via a computer network such as the internet. However, linkage can also be provided by private or inter-corporate intranets or local area networks. Sophisticated interface programming and data management are an essential part of the realization of the remote research system. The system thus is supported by advanced data-management and distributed software access, advantageously by web-based solutions. Further, another advantageous feature are cameras, most particularly web cameras, to provide real-time visual access for the remotely located researchers. The research system of the invention also provides for a 24 hour seven days a week access to the laboratory site. For certain types of tests and experiments, this is a very useful or even essential feature as these tests and experiments require continuous attention. The employment of cameras also increases the confidence into the system according to the invention and will intuitively make the remote scientist part or even master of the local, i.e. central process. The system of the invention now provides for access around the clock for researchers spread around the world, enabling to design and perform highly sophisticated and complex tests.

Tests in combinatorial materials research are usually performed with the aid of laboratory robots. Actions of these robots are transmitted by the cameras of the system according to the invention to any remotely located research site where operators can control and view the robot actions. However, as the robot performance usually is at a very high quality standard, it is advantageous to implement one or several warning elements to provide a warning signal in case of an unexpected test incident. A (visual) connection between the central laboratory site and the remotely located researcher or operator would only be built up in case such a warning element would emit a warning signal (e.g. when a reaction tends to get out of control or parameters leave their expected limits), allowing to view the status of the test and, if necessary, to take appropriate action by interfering or stopping the test.

However, although these are very advantageous features of the invention, it proves to be even more advantageous for remotely located researchers to be able to plan and design a test to be performed at the central laboratory site without having to travel which results in a great gain of valuable time. In CMR, this allows the experts to design an experiment from scratch or select appropriate ingredients to be combined or define process conditions or parameter spaces.

### Description of the Drawings

The detailed description will refer to the following drawing figures in which like reference numerals refer to like objects. It is understood that the description is in no way limitative to the scope of the present invention and merely is an illustration of a preferred embodiment of the invention. Also, the described combination of the features of the invention is not be understood as a limitation, and all the features can be combined in other constellations without departing from the spirit of the invention. In the drawings,
Figure 1 is a schematic diagram showing the principles of the invention.
Figure 2 is a schematic diagram of the overall architecture of the remote research system of the invention.
Figure 3 is a block diagram showing in a more detailed way the architecture of the remote research system of the invention.

### Detailed Description

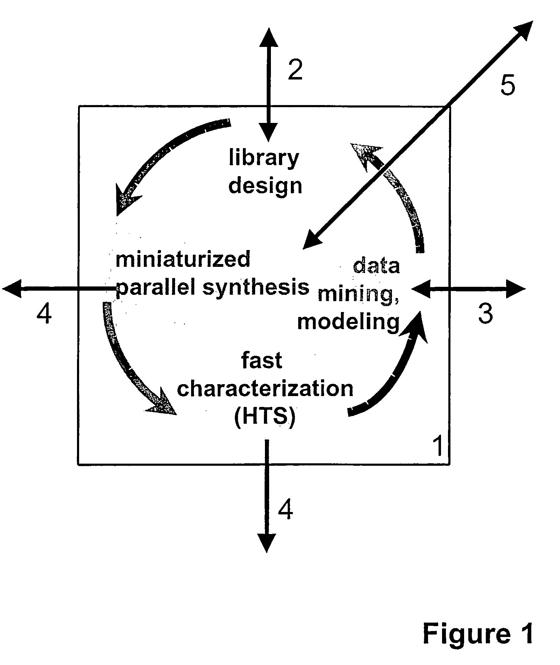

The invention is hereinafter described by ways of a remote CMR system apt to provide access for remotely located researchers to a central combinatorial materials research site. Figure 1 shows the principles of CMR in relation with the present invention. Figure 1 shows a so-called combinatorial loop. The circle within the square 1 is the inner CMR circle and visualizes the experimental hardware and computer hardware and databases located at the central research facility. Arrows 2 to 5 showing across square 1 from or to the inner CMR circle visualize the communication with remotely located sites. These sites can be other research facilities or affiliated companies or co-operation companies or even customer companies. Access for remote sites is pre-defined via access control and can be restricted, which can be, for example, temporary access.

Arrow 2 visualizes a bidirectional communication with regard to library design. Core of any CMR process is a comprehensive library data base with materials data. These data very often result from previous experiments. Design of an experiment departs from library data. In order to be able to plan and design an experiment, a remotely located researcher is given access to the library data base. This access is bidirectional as it is also essential for future experiments that new data will be accessible.

Arrows 4 show unidirectional communication. This is the case when data flow is directed towards the remotely located researcher only, for example in the case of web cameras observing laboratory activities and detectors providing experiment data for process control. The part of the CMR circle shown in the left part of Figure 1 relates to chemistry and robotics and would be referred to as the actual experiment being implemented and performed.

Arrow 3 shows bidirectional communication (data flow) with regard to data mining and modelling. This concerns information on the status of processes, the visualization of designs, processes, experimental results, data evaluation and mining, automated new designs as result of mining activities, reporting and patenting. The process of data mining and modelling is a step after the performance of an experiment in order to evaluate and interpret experimental results. In this stage, physics and data mining are the most important aspects of the activities. With the system of the invention, this stage provides hitherto unknown possibilities in evaluating test results by ways of a world wide experts discussion round.

Arrow 5 finally shows a further bidirectional communication and stands for the general possibility of the remotely located researcher to interrupt, change, reprioritize or cancel processes from his remote location.

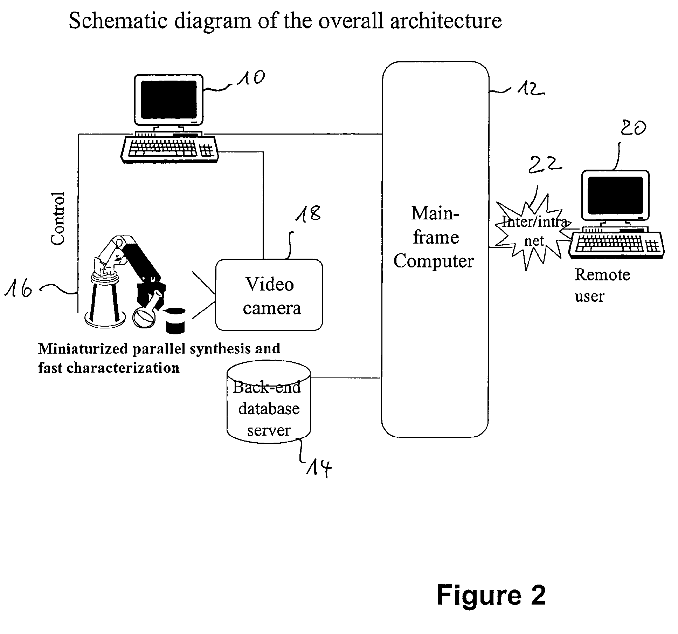

Figure 2 shows a schematic diagram of the overall architecture of the remote CMR system of the invention. A work station 10 is connected to a main frame computer (or central processing unit) 12. A backend data base server 14 for library data and other data relevant to the chemical research experiments to be performed is also connected to CPU 12. Laboratory hardware and robots 16 are linked to work station 10 for control purposes. Video cameras 18 are provided to observe the laboratory hardware during experiments, and are also linked to work station 10. Further, a remote user work station 20 is provided and connected to CPU 12 via a computer network 22 (which is the internet or an intranet or an extranet or any other netware).

As already described above, a remotely located researcher working at remote user work station 20 is able to log into the central laboratory site comprising CPU 12, library 14, work station 10, hardware 16 and cameras 18 in order to participate in the design and performance of experiments. Remote user work station 20 can be located at any part of the world. Particularly, with appropriate computer hardware equipment, it is possible for a scientist to log into the central research site when travelling. The scientist is able to participate in the experiment as if he were on site, controlling the laboratory hardware and the cameras with his computer and appropriate equipment such as a joystick etc.

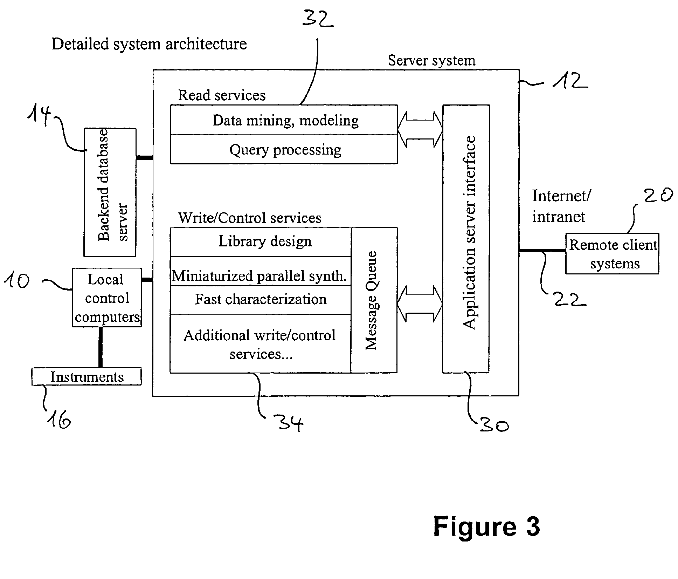

Figure 3 shows a more detailed view of the system architecture according to the invention in which the server system (CPU) 12 is illustrated in more detail. The server system 12 accordingly comprises an application server interface 30 which communicates with various solutions of the research system. In particular, these solutions comprise services such as read services 32 and write/control services 34. Read services 32 enable the user to perform tasks and assist relating to data mining and modelling as well as processing of queries. Write/control services 34 relate to library design, miniaturized parallel synthesis (a central item in CMR), fast characterization, and additional write and control services regarding control of the robots, instruments and cameras and the updating of experimental data and results and interpretations of various test.

According to the invention, design of experiments can be done remotely. For this purpose, the content of central data base 14 is accessible to the remote expert via a web-based application server, for example. As a result, the expert has all data on hand which is necessary to design the experiment with his design software, e.g. he can develop new formulations out of a fixed set of ingredients or new catalysts out of a fixed set of ligands. Further, all previous test designs can be visualized on demand and serve as a basis for the current experiment design. The design can be varied, taking into account all boundary conditions until the final design is achieved. Then, the remote expert passes his design on to the central server 12 where it is put into the waiting queue of the scheduler program governing and controlling the combinatorial process with all the hardware, considering any selected priority.

The information resulting of the experiment is stored in data bases for further interpretation. Chemical structures, recipes, processes and characterization results can be visualized by means of data base queries. Raw data, e.g. spectra and images, can be further processed to gain reduced parameters. Remote access for interpretation as offered by the system of the invention integrates the remote researcher and makes him part of the local/central project. Or, it makes him independent of the local scientists as he has round the clock access and does not depend on time zone differences. Video and/or phone conferences will become much more effective and productive because all of the participants have the full set of data available. Ideas can be discussed interactively without having to wait for several days or even weeks for preparing required diagrams etc. Thus, what has been a serious bottleneck problem of research projects is now eliminated by the system according to the invention.

The remote research system according to the invention opens various possibilities in chemical research. For example, it promotes "research on demand" and "pay-research". Particularly in the field of CMR, the features and advantages described above are essential for the improvement of quality and speed of CMR experiments as CMR consists of rather unconventional experiments with increased capacity what requires better and more effective surveillance.

Further to the advantages in research projects outlined above, the remote research system according to the invention provides for improved customer relation management possibilities. Sales and marketing representatives of a company can provide their (remote) customers with restricted access to the remote research system, enabling the customer to follow running experiments either visually or through online data base queries.

The terms and descriptions used herein are set forth by way of illustration only and are not meant as limitations. Those skilled in the art will recognize that many variations are possible within the spirit and scope of the invention as defined in the following claims, and their equivalents, in which all terms are to be understood in their broadest possible sense unless otherwise indicated. In particular, the description provided above uses the specific example of a remote CMR system. One of ordinary skill in the art will recognize that the architecture for managing and handling distributed research activities would apply equally well to any other research field.

## Claims

1. A remote research system, comprising:
a central laboratory site equipped to be used for chemical research tests;
one or several laboratory robots for performing chemical research tests;
a central processing unit (CPU);
one or several workstations linked to said central processing unit and set up to control said laboratory robots;
one or several cameras for detecting actions of said laboratory robots when performing one of said chemical research tests, said one or several cameras being linked to said central processing unit via said one or several workstations;
the remote research system further comprising:
one or several remote user workstations linked to said central processing unit via a computer network and set up to allow remote user access to chemical research tests performed in said central laboratory site, the execution of a test performed being transmitted by said one or several cameras via said central processing unit and said computer network to said one or several remote user workstations.

2. The remote research system of claim 1, wherein the chemical research test performed is a combinatorial materials research test.

3. The remote research system of claim 1, further comprising a database for storing data relevant to said chemical research tests, said database being connected to said central processing unit

4. The remote research system of claim 1, further comprising detector elements for automatically detecting results of said chemical research tests.

5. The remote research system of claim 4, wherein the remote user workstations are set up to allow remotely located researchers access to any stage of a chemical research test.

6. The remote research system of claim 5, wherein said stage of a chemical research test is the conception of said research test.

7. The remote research system of claim 5, wherein said stage of a chemical research test is the design of said research test.

8. The remote research system of claim 5, wherein said stage of a chemical research test is the evaluation of said research test.

9. The remote research system of claim 5, wherein said stage of a chemical research test is the interpretation of said research test.

10. The remote research system of claim 5, further comprising one or several warning elements to provide a warning signal in case of an unexpected test incident.

11. The remote research system of claim 10, wherein a connection between said central processing unit and said remote user workstation is only built up upon said one or several warning elements emitting a warning signal.

12. The remote research system of claim 1, wherein said computer network is the internet.

13. The remote research system of claim 1, wherein said computer network is an intranet.

14. The remote research system of claim 1, wherein said computer network is a local area network.

15. A remote research system, comprising:
a central laboratory site equipped to be used for chemical research tests;
a central processing unit (CPU);
one or several workstations linked to said central processing unit and set up to control said chemical research tests;
a database for storing data relevant to said chemical research tests, said database being connected to said central processing unit;
detector elements for automatically detecting results of said chemical research tests;
the remote research system further comprising:
one or several remote user workstations linked to said central processing unit via a computer network and set up to allow remote user access to chemical research tests performed in said central laboratory site, enabling remotely located researchers using said remote user workstations to participate remotely in any stage of the performance one of said chemical research tests.

16. The remote research system of claim 15, further comprising one or several laboratory robots for performing chemical research tests.

17. The remote research system of claim 16, wherein the chemical research test performed is a combinatorial materials research test.

18. The remote research system of claim 16, further comprising one or several cameras for providing visual control over a performed research test, said one or several cameras being linked to said central processing unit via said one or several workstations.

19. The remote research system of claim 18, wherein said one or several cameras transmit actions of said laboratory robots when performing one of said chemical research tests.

20. The remote research system of claim 18, further comprising the execution of a test performed being transmitted by said one or several cameras via said central processing unit and said computer network to said one or several remote user workstations.

21. The remote research system of claim 20, further comprising a control element enabling said remotely located researcher to control the field of vision of said one or several cameras.

22. The remote research system of claim 18, wherein said one or several cameras are web cameras.

23. The remote research system of claim 18, wherein said computer network is the internet.

24. The remote research system of claim 18, wherein said computer network is an intranet.

25. The remote research system of claim 18, wherein said computer network is a local area network.

26. The remote research system of claim 18, further comprising one or several warning elements to provide a warning signal in case of an unexpected test incident.

27. The remote research system of claim 26, wherein a connection between said central processing unit and said remote user workstation is only built up upon said one or several warning elements emitting a warning signal.

28. The remote research system of claim 18, wherein said stage of a chemical research test is the conception of said research test.

29. The remote research system of claim 18, wherein said stage of a chemical research test is the design of said research test.

30. The remote research system of claim 18, wherein said stage of a chemical research test is the evaluation of said research test.

31. The remote research system of claim 18, wherein said stage of a chemical research test is the interpretation of said research test.

32. The remote research system of claim 18, wherein the remote research system is set up to enable said remotely located researcher to interfere with a chemical research test in progress.

33. The remote research system of claim 18, further comprising software tools enabling said remotely located researcher to perform data mining queries in said database.

34. The remote research system of claim 18, further comprising software tools enabling said remotely located researcher to perform archiving processes in said database.

35. A computer system to perform remote chemical research, comprising:
a central server system;
a backend database server connected to said central server system;
one or several local control computers connected to said central server system and providing access to said central server system for local users;
chemical laboratory equipment connected to said one or several local control computers;
one or several remote client systems connected to said central server system via a computer network and providing access to said central server system for remotely located users;
said central server system further comprising an application server interface for connecting local users and remotely located users to research test services provided by said central server system, said research test services being database services and conceptional services.

36. The computer system of claim 35, wherein said conceptional services comprise the designing of a chemical research test, the control of a chemical research test and the evaluation and interpretation of a chemical research test.

37. The computer system of claim 36, wherein said conceptional services further comprise the designing and updating of a library stored in said database.

38. The computer system of claim 35, wherein said database services comprise the archiving of data in said database.

39. The computer system of claim 38, wherein said database services comprise the performance of data mining in said database.
